(19) Europäisches Patentamt
European Patent Office
Office européen des brevets

(11) **EP 2 447 257 A1**

(12) **EUROPEAN PATENT APPLICATION**
published in accordance with Art. 153(4) EPC

(43) Date of publication:
**02.05.2012 Bulletin 2012/18**

(21) Application number: **10791860.9**

(22) Date of filing: **23.06.2010**

(51) Int Cl.:
*C07D 311/62* (2006.01)  *A61K 31/352* (2006.01)
*A61P 9/10* (2006.01)  *A61P 43/00* (2006.01)

(86) International application number:
**PCT/JP2010/004177**

(87) International publication number:
**WO 2010/150538 (29.12.2010 Gazette 2010/52)**

(84) Designated Contracting States:
**AL AT BE BG CH CY CZ DE DK EE ES FI FR GB
GR HR HU IE IS IT LI LT LU LV MC MK MT NL NO
PL PT RO SE SI SK SM TR**

(30) Priority: **23.06.2009 JP 2009148658**

(71) Applicant: **National Cerebral and Cardiovascular
Center
Suita-shi
Osaka 565-8565 (JP)**

(72) Inventors:
• **NISHIZUKA, Taichi
Moriya-shi
Ibaraki 302-0106 (JP)**
• **SAWAMURA, Tatsuya
Suita-shi
Osaka 565-8565 (JP)**

(74) Representative: **Dossmann, Gérard
Casalonga & Partners
Bayerstrasse 71-73
80335 München (DE)**

(54) **DRUG FOR INHIBITING LECTIN-LIKE OXIDIZED LDL RECEPTOR AND DRUG FOR
PREVENTING ARTERIOSCLEROSIS**

(57)    A compound having an excellent activity to inhibit a lectin-like oxidized LDL receptor has been identified.

There are provided a drug for inhibiting a lectin-like oxidized LDL receptor **characterized by** including a trimeric or higher polymeric procyanidin as an active ingredient, which is useful in treating and preventing diseases, in particular, arteriosclerotic diseases, for which a lectin-like oxidized LDL receptor acts as an aggravating factor;

the aforementioned drug for inhibiting a lectin-like oxidized LDL receptor, **characterized in that** the procyanidin is derived from a plant material selected from the group consisting of apples, grape seeds, peanut seed coats and pine barks; and
a drug for preventing arteriosclerosis including a trimeric or higher polymeric procyanidins as an active ingredient, and **characterized by** having an activity to inhibit a lectin-like oxidized LDL receptor.

**Description**

[Technical Field]

The present invention relates to an inhibitory drug that inhibits a bonding between a lectin-like oxidized LDL receptor and an oxidized LDL, and also to a drug for preventing arteriosclerosis which contains the inhibitory drug.

In recent years, the number of patients suffering from various lifestyle-related diseases has increased in developed countries including Japan due to the changes in living environment and changes in eating habits. Major lifestyle-related diseases include diabetes, hypertension and hyperlipidemia , and the number of people who developed these diseases has been increasing. In addition, the number of people, even if they do not develop severe symptoms, who show signs of symptoms (so called "those at risk") has also shown an increasing trend. Further, these lifestyle-related diseases often cause serious illness.

Priority is claimed on Japanese Patent Application No. 2009-148658, filed June 23, 2009, the content of which is incorporated herein by reference.

[Background Art]

An arteriosclerosis disease is one of the diseases caused by lifestyle-related diseases, and hyperlipidemia, especially hypercholesterolemia, has been regarded as a major cause. Currently, drugs and foods for specified health use for the sake of preventing or treating cardiovascular diseases such as arteriosclerotic diseases have been certified, using the effect of lowering the concentrations (levels) of cholesterol and neutral fat in the blood as an indicator. For example, statin serving as a cholesterol synthesis inhibitor and peroxisomal proliferator-activated receptor (PPAR) agonists which reduce the neutral fat levels in the blood are effective to cardiovascular diseases such as ischemic heart disease, and thus have been widely used.

Since the arteriosclerotic diseases and the lifestyle-related diseases acting as the major cause therefor are chronic diseases, an effective prophylactic agent which can be administered safely for a long period of time has been desired for effective prevention. For example, as a drug effective for the prevention of arteriosclerosis, an adiponectin regulator characterized by containing a polyphenol derived from apples as an active ingredient has been disclosed (for example, refer to Patent Document 1). Adiponectin was discovered as a secreted protein specifically expressed at high levels in fat cells. However, it has become clear from the later studies that it has anti-arteriosclerotic effects to prevent adhesion of monocytes to vascular endothelial cells, proliferation of smooth muscle cells, or the like, and the anti-arteriosclerotic effects can be achieved by controlling the level of adiponectin in the blood.

Various activities and effects of polyphenols derived from apples have been reported, including the activities and effects related to lipid metabolism. For example, procyanidins contained in the polyphenols derived from apples have been reported to inhibit pancreatic lipase, thereby suppressing the absorption of triglycerides (for example, refer to Non-Patent Document 1).

In addition, oxidized low-density lipoproteins (LDL) produced by the oxidation of LDLs have been found to promote arteriosclerosis, and the anti-arteriosclerotic effects can be achieved by reducing the level of oxidized LDLs in the blood. For example, an arteriosclerosis prophylactic agent having pine bark extracts that contain proanthocyanidins at a ratio of less than 95% by weight in terms of dry weight as active ingredients has been disclosed (for example, refer to Patent Document 2). Pine bark extracts have a high inhibitory effect on lipid oxidation in blood vessels, and thus it is possible to reduce the level of oxidized LDLs in the blood by taking pine bark extracts.

On the other hand, it is thought that the action by the oxidized LDL to promote arteriosclerosis is caused due to the incorporation of the oxidized LDL into vascular endothelial cells via a lectin-like oxidized LDL receptor (LOX-1; Lectin-like oxidized low-density lipoprotein receptor-1). LOX-1 was originally identified as an oxidized LDL receptor in vascular endothelial cells. However, it is now known as a factor that promotes cardiovascular diseases, such as inflammation, arteriosclerosis, thrombosis, myocardial infarction, and vascular restenosis following a catheter treatment. In other words, the anti-arteriosclerotic effects can also be achieved by inhibiting the oxidized LDL from binding to LOX-1 and being incorporated into vascular endothelial cells.

As prophylactic agents that exhibit an activity to inhibit LOX-1 as described above, a composition containing plant extracts of bracken (*Pteridium aquilinum*) that belongs to Dennstaedtiaceae as active ingredients and exhibiting a LOX-1 antagonist activity (for example, refer to Patent Document 3), an arteriosclerosis inhibitor containing extracts of plants that belong to Rutaceae as active ingredients and exhibiting an LOX-1 antagonist activity (for example, refer to Patent Document 4) and the like have been disclosed.

[Citation List]

[Patent Documents]

[Patent Document 1] Japanese Unexamined Patent Application, First Publication No. 2006-193502
[Patent Document 2] Japanese Unexamined Patent Application, First Publication No. 2005-23032
[Patent Document 3] Japanese Unexamined Patent Application, First Publication No.2007-297381
[Patent Document 4] Japanese Unexamined Patent Application, First Publication No. 2007-320956

[Non-Patent Document]

[Non-Patent Document 1] Sugiyama and 6 others, Journal of Agricultural and Food Chemistry, 2007, Volume 55, pp. 4604-4609.

[Summary of Invention]

[Technical Problem]

As with statin and other agents, the prophylactic agents described in Patent Documents 1 and 2 are intended to prevent arteriosclerosis by suppressing the blood lipid levels. On the other hand, the prophylactic agents described in Patent Documents 3 and 4 are intended to block the site of action in the arteriosclerosis-inducing lipoproteins such as oxidized LDL for preventive therapy through an entirely new mechanism.
However, the effects by these prophylactic agents to inhibit the oxidized LDL from binding to LOX-1 and being incorporated into vascular endothelial cells cannot be said to be satisfactory, and there has been an increasing demand for prophylactic agents that are more effective to arteriosclerosis.

The present invention has been developed in light of the circumstances described above, and has an object of identifying a compound having an excellent activity to inhibit lectin-like oxidized LDL receptors and providing a drug useful in treating and/or preventing diseases, especially arteriosclerotic diseases, where LOX-1 acts as an aggravating factor.

It should be noted that in the present invention and in the description of the present application, the phrase "activity to inhibit lectin-like oxidized LDL receptors" refers to the activities to inhibit the bonding between LOX-1 and oxidized LDL and to inhibit the intracellular uptake of oxidized LDL.

[Solution to Problem]

Based on the prior arts described above, the present inventors searched for those exhibiting even higher inhibitory effects on the lectin-like oxidized LDL receptors, and completed the present invention, as a result, by discovering that proanthocyanidins, especially those that are trimers or higher polymers, exhibit potent effects.

That is, the present invention provides the following aspects:

(1) A drug for inhibiting a lectin-like oxidized LDL receptor, characterized by including, as an active ingredient, a trimeric or higher polymeric procyanidin;
(2) The drug for inhibiting a lectin-like oxidized LDL receptor according to the above aspect (1), **characterized in that** the procyanidin is derived from a plant material selected from the group consisting of apples, grape seeds, peanut seed coats and pine barks;
(3) A drug for preventing arteriosclerosis characterized by containing, as an active ingredient, a trimeric or higher polymeric procyanidin, and having an activity to inhibit lectin-like oxidized LDL receptors; and
(4) The drug for preventing arteriosclerosis according to the above aspect (3), **characterized in that** the procyanidin is derived from a plant material selected from the group consisting of apples, grape seeds, peanut seed coats and pine barks.

[Advantageous Effects of Invention]

The drug for inhibiting lectin-like oxidized LDL receptors and the drug for preventing arteriosclerosis, which employs the former drug, according to the present invention are capable of inhibiting the binding of oxidized LDL to lectin-like oxidized LDL receptors and effectively suppressing the intracellular uptake of oxidized LDL. In addition, procyanidin serving as an active ingredient in the drug for inhibiting lectin-like oxidized LDL receptors according to the present invention and the like is a compound conventionally contained in foods and beverages, and is a highly safe compound with which almost no side effects have been reported even when administered for a long period of time. For this reason, through continuous ingestion of the drug for inhibiting lectin-like oxidized LDL receptors according to the present invention and the like, it can be expected to effectively prevent the arteriosclerosis disease and to reduce the risk of suffering from the disease.

[Brief Description of Drawings]

FIG. 1 is a diagram showing the results of absorbance at 450 nm (OD450) for procyanidins with each degree of polymerization measured in Example 1.

FIG. 2 is a diagram showing the calculated uptake rate (%) of oxidized LDL for procyanidins with each degree of polymerization in Example 2.

FIG. 3 is a diagram showing the uptake rate (%) of oxidized LDL when the respective polyphenol was added in Example 3.

FIG. 4 is a diagram showing the binding rate (%) of oxidized LDL when various procyanidins were added in Example 4.

FIG. 5 is a diagram showing the binding rate (%) of oxidized LDL when four types of trimeric procyanidins with different structures were added in Example 5.

FIG. 6 are diagrams showing a stained image (A) of lipids in the mesenteric arteries of rats orally administered with apple polyphenols (AP), and the number of lipid deposits observed in the mesenteric artery (B), in Example 7.

FIG. 7 is a diagram showing the level of oxidized LDL in the blood of rats orally administered with AP in Example 7.

[Description of Embodiments]

Procyanidins are compounds having a structure in which Flavan-3-ol is condensed repeatedly between the carbon atoms from the 4th to 8th positions or from the 4th to 6th positions, and a typical procyanidin has a structure in which (+)-catechin and (-)-epicatechin are condensed repeatedly between the carbon atoms from the 4th to 8th positions or from the 4th to 6th positions. Procyanidins may adopt complex structures, depending on the difference between the binding positions (i.e., whether Flavan-3-ol binds to the 6th position or to the 8th position), the difference between the type of bound isomers (i.e., the type of Flavan-3-ol to be bound), and also the resulting conformation of the monomers bound thereto.

The inventors of the present invention have screened for compounds having an activity to inhibit lectin-like oxidized LDL receptors (hereafter, referred to as the "LOX-1 inhibiting activity") from amongst about 500 kinds of food materials, using an enzyme-linked immunosorbent assay (ELISA) for measuring the bonding between the human LOX-1 protein and the oxidized LDL. The ELISA-based method was conducted by modifying the method of Sato and others (refer to Atherosclerosis, 2008, vol. 200, No. 2, pages 303-309). As a result, apple polyphenols were found to have an LOX-1 inhibiting activity. In addition, it was also newly discovered that when the oxidized LDL is reacted with the cells that are expressing LOX-1 in the cell surface in the presence of apple polyphenols, the bonding between LOX-1 and oxidized LDL is inhibited and consequently the intracellular uptake of oxidized LDL is also inhibited; i.e., apple polyphenols have an LOX-1 inhibiting activity. Moreover, it was also found that this LOX-1 inhibiting activity by the apple polyphenols was observed more strongly in procyanidins mainly, and especially in trimeric or higher polymeric procyanidins.

The drug for inhibiting a lectin-like oxidized LDL receptor of the present invention (hereafter referred to as the "drug for inhibiting LOX-1") is characterized by containing, as an active ingredient, a trimeric or higher polymeric procyanidins. The LOX-1 inhibiting activity associated with procyanidins depends on the degree of polymerization of procyanidins, and procyanidins with a higher degree of polymerization exhibit higher LOX-1 inhibiting activities. The drug for inhibiting LOX-1 according to the present invention is capable of exhibiting the LOX-1 inhibiting activity which is markedly superior to those of procyanidins whose degree of polymerization is two or less (namely, dimeric or monomeric procyanidins) or procyanidins that are not purified into fractions based on the degree of polymerization, by containing, as an active ingredient, a relatively large procyanidin whose degree of polymerization is 3 or more. It should be noted that the dependency of LOX-1 inhibiting activity associated with procyanidins on the degree of polymerization of procyanidins is a discovery made by the inventors of the present invention for the first time.

The drug for inhibiting LOX-1 according to the present invention may be a drug that contains only procyanidins of a specific degree of polymerization as active ingredients or a drug that contains a mixture of several types of procyanidins with different polymerization degrees as an active ingredient. With respect to the drug for inhibiting LOX-1 according to the present invention, it is preferable to use all procyanidins with a degree of polymerization of 3 or more as active ingredients (namely, except for monomeric and dimeric procyanidins), amongst the procyanidins which have been extracted and purified from the plant sources described later.

It should be noted that in the description of the present application, the term "monomeric procyanidins" refers to the catechins (including catechins and epicatechins).

In addition, the active ingredients of the drug for inhibiting LOX-1 according to the present invention are not limited to the compounds with a specific structure, as long as they are procyanidins with a polymerization degree of 3 or more. This is because procyanidins with any type of structures would exhibit LOX-1 inhibiting activity as long as their degree of polymerization is 3 or more. It should be noted that because the procyanidins having a structure in which at least one condensation site is present between the carbon atoms from the 4th to 6th positions of Flavan-3-ol or the procyanidins terminated with epicatechin exhibit relatively strong LOX-1 inhibiting activity, it is preferable to include procyanidins having these structures as active ingredients.

Trimeric or higher polymeric procyanidin may be procyanidins synthesized by known organic synthesis methods, or natural procyanidins may also be used, which are prepared by using the plants containing procyanidins as source materials and obtaining procyanidin fractions from these plant sources, followed by purification based on the degree of polymerization. Natural procyanidins can be prepared, for example, by obtaining a procyanidin fraction containing procyanidins from the extracts extracted from the plant sources, followed by the purification of procyanidins having a polymerization degree of 3 or more from this procyanidin fraction through column chromatography or the like.

Examples of the plant sources include apples (Rosaceae), mangosteens (Guttiferae), olives (Oleaceae), grapevines (Vitaceae), cranberries and cowberries (Ericaceae), pomegranates (Punicaceae), blackcurrants (Saxifragaceae), cacaos (Sterculiaceae), soybeans (black beans) and groundnuts (peanuts) (Fabaceae), and bark of pines or the like. Of these plants, tissues with a relatively high procyanidin content or tissues that are easy to extract can be used as source materials. As an active ingredient of the drug for inhibiting LOX-1 according to the present invention, procyanidins derived from apples, grapes, peanuts, pines or the like are preferred, and procyanidins extracted and purified from apple fruits, grape seeds, peanut seed coats and pine barks are more preferred. Of these, procyanidins derived from the fruits of apples are particularly desirable.

Extraction of procyanidins from the plant sources can be carried out through conventional methods with no particular limitations as long as the method enables the extraction of procyanidins from the plants without causing damages thereto. For example, extracts containing procyanidins can be prepared by collecting tissues such as fruits, seeds, leaves, stems and bark from the plant sources, and after an appropriate processing such as cutting and crushing, conducting extraction with heating in water or in an organic solvent such as alcohols. When an extraction is conducted using an organic solvent, the organic solvent may be removed from the extract by distillation or the like. Also, in the case of fruits such as apples, the fruit juice obtained by squeezing the fruit materials through conventional methods can also be used as an extract. It should be noted that although the squeezed fruit juice and the extracts from which organic solvents have been removed can be used directly as they are, it is preferable to use clear fruit juice or extracts that have undergone processes such as centrifugation and filtration.

The procyanidin fraction is obtained by conventional methods from the extracts obtained in this manner. The procyanidin fraction may be prepared through a one-step purification procedure or may be prepared by carrying out a multi-step purification procedure. For example, a polyphenol fraction is separated and purified from the extract obtained from the plant sources using an adsorbent or the like which may adsorb polyphenols, and the procyanidin fraction can be purified from the obtained polyphenol fraction obtained by employing an adsorption chromatography method or the like.

Examples of the adsorbents which may adsorb polyphenols include adsorbents such as ion exchange resins, synthetic adsorption resins and silica gel, and gel filtration agents. Adsorption and elution of the polyphenols in the extract to adsorbents can be carried out by conventional methods. For example, the polyphenols can be adsorbed by passing a liquid extract obtained from plant sources through a column filled with an adsorbent. Note that pH of the liquid extract is adjusted to between 4 and 9, preferably 5 and 7, and more preferably 5 and 6, before passing the liquid through the column. Subsequently, a sufficient amount of pure water or deionized water (at pH of 5 to 7) is made to pass through the column to remove non-adsorptive materials (sugars, organic acids and the like) in the column. Thereafter, polyphenols can be eluted with an alcohol solvent, for example, a 10 to 90% (and preferably 30 to 80%) ethanol. The polyphenol fraction obtained in this manner is mainly composed of procyanidins, and also contains polyphenols including the derivatives (esters) of caffeic acids such as chlorogenic acid, p-coumaric acid derivatives, flavan-3-ols (catechins), flavonols such as quercetin glycosides and chalcones such as phloretin glycosides.

As a column stationary phase in the adsorption chromatography employed for the separation and purification of procyanidin fractions from the polyphenol fraction, those that are similar to the adsorbent described above can be used. Note that pH of the polyphenol fraction is adjusted to between 4 and 9, preferably 5 and 7, and more preferably 5 and 6, before passing the liquid through the column. In addition, prior to elution, the column is preferably washed thoroughly with pure water or deionized water (at a pH of 5 to 7). Moreover, as an elution solvent (mobile phase), any solvent may be used as long as it is a solvent capable of sufficiently differentiate the elution time of procyanidins from the column stationary phase and the elution time of polyphenols excluding procyanidins, and can be determined appropriately in consideration of the type of column stationary phase, the equipment to be used, and so on.

For example, by subjecting the polyphenol fraction dissolved in methanol to a binary gradient elution by adsorption chromatography using silica gel as a column stationary phase and hexane-methanol-acetone as an elution solvent, the polyphenols excluding procyanidins can be eluted prior to procyanidins, and also procyanidins can be sequentially eluted starting from those with smaller polymerization degrees (for example, refer to Non-Patent Document 1). Accordingly, by examining the elution time for procyanidins with each degree of polymerization in advance using standards and the like, procyanidin fractions containing only the procyanidins with a desired degree of polymerization can be obtained. In addition, by collecting all the eluted fractions from that of the trimeric procyanidins onwards, the fraction containing all procyanidins whose degree of polymerization is 3 or higher can also be obtained. Alternatively, for example, by collecting, concentrating and freeze-drying the eluted fractions from that of the dimeric procyanidins onwards, followed by their redissolution in methanol and separation by adsorption chromatography in the same manner, the fractions of procyanidins with desired degree of polymerization can be recovered in a state of higher purification degrees.

As an active ingredient of the drug for inhibiting LOX-1 according to the present invention, the procyanidin fraction obtained by the above-mentioned methods may be used directly as it is, or a concentrated liquid prepared by concentrating the procyanidin fraction may also be used. For example, by concentrating under reduced pressure at 25 to 100°C, preferably at 35 to 90°C, solvents such as methanol can be removed from the procyanidin fraction for concentration. Moreover, a powder may also be used, which is prepared by concentrating the obtained procyanidin fraction, followed by spray drying or freeze-drying the obtained concentrated liquid, either directly as it is or adding an auxiliary agent powder such as dextrin thereto. Furthermore, procyanidins dissolved in a preferred solvent may also be used by dissolving a procyanidin powder in an organic solvent such as ethanol or in an organic acid such as citric acid. Note that as a solvent for dissolving procyanidins, for example, an organic solvent such as ethanol, an organic acid such as citric acid, or the like can be used.

The amount of trimeric or higher polymeric procyanidins contained in the drug for inhibiting LOX-1 according to the present invention is not particularly limited as long as the amount is sufficient for achieving the LOX-1 inhibiting activity, and can be determined appropriately in consideration of the purity, dosage form or administration method of procyanidins, and the like. In the present invention, the proportion of trimeric or higher polymeric procyanidins contained in the drug for inhibiting LOX-1 is preferably 50% or higher, more preferably 80% or higher, and still more preferably 100%, since the LOX-1 inhibiting activity can be achieved more satisfactorily.

In addition, the drug for inhibiting LOX-1 according to the present invention may also contain other components as long as they do not impair the LOX-1 inhibiting activity by trimeric or higher polymeric procyanidins. For example, auxiliary materials or additives that are commonly used, such as excipients, stabilizers, preservatives and viscosity modifiers, can be contained.

Since the drug for inhibiting LOX-1 according to the present invention provides an excellent LOX-1 inhibiting activity when administered, it is preferable to be added as an active ingredient for drugs to be taken in order to treat or prevent diseases, where LOX acts as an aggravating factor, such as inflammation, arteriosclerosis, thrombosis, myocardial infarction, and vascular restenosis following catheter treatments. In particular, by including the drug for inhibiting LOX-1 according to the present invention as an active ingredient, that is, by including trimeric or higher polymeric procyanidins as an active ingredient, a drug for preventing arteriosclerosis which is highly safe and also exhibits high preventive effects can be produced.

A drug for preventing arteriosclerosis containing the drug for inhibiting LOX-1 according to the present invention as an active ingredient (hereafter, referred to as a drug for preventing arteriosclerosis according to the present invention) can be prepared by conventional methods. In addition, auxiliary materials or additives that are commonly used can be added upon the production of these drugs. Examples of such materials and additives include glucose, fructose, sucrose, maltose, sorbitol, stevioside, rubusoside, corn syrup, lactose, L-ascorbic acid, d1-α-tocopherol, sodium erythorbate, glycerin, propylene glycol, glycerin fatty acid esters, sucrose fatty acid esters, sorbitan fatty acid esters, gum arabic, carrageenan, casein, gelatin, pectin, agar, vitamin C, B-complex vitamins, vitamin E, nicotinamide, calcium pantothenate, amino acids, calcium salts, surfactants, pigments, perfumes and preservatives.

Further, there are no particular limitations on the dosage form for the drug for inhibiting LOX-1 according to the present invention or the drug for preventing arteriosclerosis according to the present invention as long as the dosage form achieves an LOX-1 inhibiting activity, and examples thereof include tablets, liquids, capsules, drinks and troches.

The amount of trimeric or higher polymeric procyanidins in the drug for preventing arteriosclerosis according to the present invention is not particularly limited as long as the amount is sufficient for achieving the LOX-1 inhibiting activity, and can be determined appropriately in consideration of the purity, dosage form or administration method of procyanidins, the sex, age, weight or health status of the subjects of intake, and the like.

In order to attain the effects of inhibiting LOX-1 and the effects of preventing arteriosclerosis, it is possible to include trimeric or higher polymeric procyanidins in the drug for inhibiting LOX-1 or the drug for preventing arteriosclerosis according to the present invention, so that the amount of intake of trimeric or higher polymeric procyanidins for adults per day in terms of dry weight is, for example, from 10 to 3,000 mg, and preferably from 30 to 1,000 mg. In addition, the drug for inhibiting LOX-1 or the drug for preventing arteriosclerosis according to the present invention may also be taken orally once or several times a day.

[Examples]

Next, the present invention will be described in more detail using a series of examples, although the present invention is in no way limited by the following examples.

[Preparation Example 1: Preparation of apple polyphenol (AP)]

300 kg of young apple fruits from Aomori Prefecture was crushed and squeezed to obtain 210 kg of fruit juice. Pectinase was added to the obtained fruit juice for clarification so that the concentration thereof was 30 ppm, and after centrifugation, further clarification was carried out by diatomaceous earth (silica 300S, manufactured by Chuo Silika Co., Ltd.) filtration to obtain a clarified fruit juice. The clarified fruit juice was passed through a column filled with an adsorption resin (Diaion SP-850, manufactured by Mitsubishi Chemical Corporation) to adsorb polyphenols. Subsequently, pure water was made to pass through to remove non-adsorptive materials (sugars, organic acids and the like) in the column. The polyphenols were then eluted with a 40% alcohol. The alcohol was concentrated under reduced pressure from the obtained polyphenol fraction to prepare approximately 2 kg of extracted powder (AP). The components in the extracted powder were analyzed using reverse phase high performance liquid chromatography. As a result, it was confirmed that the components consisted of chlorogenic acids (about 20% by weight), phloretin glycosides (about 5% by weight), flavonols (about 15% by weight), procyanidins (about 50% by weight), and other browning substances (about 10% by weight). In addition, as a result of analysis by matrix-assisted laser desorption/ionization - time-of-flight mass spectrometry (MALDI-TOF/MS, manufactured by Applied Biosystems), it was confirmed that these procyanidins were oligomers or polymers from dimers up to 15-mers that were constituted of flavonols such as catechins and epicatechins (refer to M. Olinishikameyama, et. al., Mass Spectrometry, 1997, vol. 11, pp. 31-36).
In those cases where AP was used in the following examples, this extracted powder of AP was suspended in the reagent grade dimethyl sulfoxide (DMSO) for biochemical analysis (manufactured by Wako Pure Chemical Industries, Ltd.) and was diluted with ultrapure water for use, where appropriate, so that the final concentration of DMSO in the reaction solution was 1% or less.

[Preparation Example 2: Preparation of procyanidins based on polymerization degree]

Procyanidins were separated and purified from the AP based on the polymerization degree in accordance with the technique described in Now-Patent Document 1 (adsorption chromatography).
First, the AP powder prepared above was dissolved in methanol, and was then applied to a column Inertsil PREP-SIL (manufactured by GL Sciences Inc.) filled with silica gel, and binary gradient elution was carried out by using hexane-methanol-acetone as an elution solvent. As a result, polyphenols other than procyanidins were eluted first, and procyanidins were then sequentially eluted starting from those with smaller polymerization degrees. All the eluted fractions from that of the dimeric procyanidins onwards were collected and this collection was defined as a procyanidin fraction. Subsequently, this procyanidin fraction was concentrated and freeze-dried, and was then dissolved in methanol once again and separated by adsorption chromatography in the same manner, so that procyanidin fractions for each degree of polymerization were collected separately. However, it should be noted that all of octameric or higher polymeric procyanidins were collected as a single fraction. These collected fractions were concentrated and freeze-dried, and were then suspended in the reagent grade DMSO for biochemical analysis (manufactured by Wako Pure Chemical Industries, Ltd.) for use. Note that when used in the following examples, this DMSO suspension was diluted with ultrapure water for use, where appropriate, so that the final concentration of DMSO in the reaction solution was 1% or less.
It should be noted that although AP was used in the present preparation example, it is possible to prepare procyanidin fractions for each degree of polymerization separately in the same manner even when polyphenols derived from other plants were used.

[Preparation Example 3: Preparation of ex-hLOX-1]

A region (from 61 to 273 base sequence) encoding an extracellular domain (ex-hLOX-1) within the human LOX-1 cDNA (Genbank: NM002543) was integrated into an expression vector pSecTag/FRT/V5His (manufactured by Invitrogen), and a base sequence encoding the IgK selection signal was further incorporated so that the signal was added to the N-terminus of ex-hLOX-1, thereby preparing a plasmid for expressing ex-hLOX-1. This plasmid was transfected into 293F cells using the FreeStyle 293 Expression System (manufactured by Invitrogen), thereby expressing ex-hLOX-1, After culturing the transfected cells for 4 days, the expressed protein ex-hLOX-1 was recovered from the culture medium using the Ni-NTA superflow cartride (manufactured by Qiagen).

[Preparation Example 4: Preparation of EL1SA plate where ex-hLOX-1 was fixed]

First, a protein solution of ex-hLOX-1 prepared in Preparation Example 3 was adjusted to a concentration of 5 $\mu$g/ml using phosphate buffered saline (PBS) and was dispensed in a 384-well EIA Plate (manufactured by Greiner, Frickenhausen, Germany, product number: 781061) at 50 $\mu$l/well. By leaving the plate to stand overnight at 4°C, ex-hLOX-1 was fixed in the wells. Then, after washing each well three times with PBS, 80 $\mu$l/well of a HEPES buffer containing 3% bovine serum albumin (BSA) was dispensed to carry out a blocking treatment. Then, each well was further washed three times with PBS to produce an ELISA plate. Note that A-7888 available from Sigma-Aldrich Co. LLC. was used as BSA, and a mixture prepared using 15630 available from GIBCO and 191-01665 available from Wako Pure Chemical Industries, Ltd. was adjusted so as to achieve concentrations of 10 mM HEPES and 150 mM NaCl and a pH of 7.0, and was used as a HEPES buffer, respectively.

[Preparation Example 5: Preparation of oxidized LDL]

First, plasma was separated and recovered from the blood of healthy individuals which was collected in a blood collection tube where a buffer containing acid-citrate-dextrose (ACD) was added. After adjusting the specific gravity to 1.019 by adding potassium bromide to the obtained plasma, a centrifugation treatment was carried out at 58,000 rpm for 20 hours. The obtained lower layer was transferred to a separate tube, and after adjusting the specific gravity to 1.063 with potassium bromide, a centrifugation treatment was carried out at 58,000 rpm for 20 hours. Note that the ultracentrifuge L-80 manufactured by Beckman Coulter, Inc. was used. The recovered upper layer was dialyzed using the Slide-A-Lyzer (registered trademark) Dialysis Cassettes 10K MWCO (manufactured by Takara Bio Inc.) and PBS as an external solution (the external solution was replaced four times) to obtain a purified human LDL.
The BCA Protein Assay Kit (manufactured by Pierce) was used to measure the amount of protein, and a solution was adjusted with PBS so that the concentration of purified human LDL was 3 mg/ml. After adding copper sulfate thereto so as to achieve a concentration of 7.5 $\mu$M, the resultant was incubated in a $CO_2$ incubator at 37°C for 16 hours. Subsequently, the resulting solution was dialyzed using a 0.15 M sodium chloride solution containing 2 mM EDTA as an external solution (the external solution was replaced four times) to obtain a human oxidized LDL.

[Preparation Example 6: Preparation of DiI-labeled oxidized LDL]

The prepared human oxidized LDL was diluted and adjusted using a 0,15 M sodium chloride solution containing 2 mM EDTA so as to achieve a concentration of 1 mg/ml. DiI (# D282, manufactured by Invitrogen) and Lipoprotein Deficient Serum (manufactured by Sigma-Aldrich Co. LLC.) were added to this human oxidized LDL solution so that the final concentrations thereof were 0.3 mg/ml and 5 mg/ml, respectively, and the reaction was allowed to proceed at 37°C for 18 hours. It should be noted that DiI was suspended in DMSO so as to achieve a concentration of 30 $\mu$g/ml and used as a solution. After the reaction, the specific gravity was adjusted to 1.15 with sodium chloride and potassium bromide, and a centrifugation treatment was then carried out at 58,000 rpm for 20 hours. The recovered upper layer was dialyzed using the Slide-A-Lyzer (registered trademark) Dialysis Cassettes 10K MWCO (manufactured by Takara Bio Inc.) and a 0.15 M sodium chloride solution containing 2 mM EDTA as an external solution (the external solution was replaced four times) to obtain a DiI-labeled oxidized LDL.

[Preparation Example 7: Preparation of TetOn hLOX-1 CHO cells]

Cultured cells in which the expression of hLOX-1 can be induced by the addition of doxycycline were prepared using the tetracycline expression controlling system (manufactured by Clontech). Note that as a medium for culturing cells, Ham's F-12+GlutaMAX (manufactured by Gibco) was used, to which fetal bovine serum (FBS) and Antibiotics-Antimicotics (manufactured by Gibco) were added so that their final concentrations were 10% and 1%, respectively.
First, a vector for expressing hLOX-1 in which the human LOX-1 cDNA (Genbank: NM002543) was integrated into the pTRE2hyg expression vector (manufactured by Clontech) was prepared. This vector for expressing hLOX-1 was transfected into CHO-K1 Tet-On cells (manufactured by Clontech) by using the Lipofectamin 2000 transfection reagent (manufactured by Invitrogen). The cells were cultured by adding hygromysin B (manufactured by Wako Pure Chemical Industries, Ltd.) and G418 (manufactured by Calbiochem) to the culture medium so that their final concentrations were 400 $\mu$g/ml and 100 $\mu$g/ml, respectively, to select the cells in which the vector for expressing hLOX-1 was introduced. These cells were used as the TetOn hLOX-1 CHO cells.

[Preparation Example 8: Preparation of anti-LOX-1 antibody]

An anti-LOX-1 antibody was produced in accordance with the method of Sawamura and others (refer to Nature, 1997, vol. 386, pp. 73-77).

In other words, the CHO cells expressing hLOX-1 were treated with 5 mM EDTA-PBS (at room temperature for 5 minutes), and then suspended in a buffer containing protease inhibitors [consisted of 25 mM of HEPES (pH 7.4), 10 mM of magnesium chloride, 0.25 M of sucrose, and protease inhibitors (including 10 U/mL of Aprotinine, 2 $\mu$g/mL ofPepstatin, 50 $\mu$g/mL of Leupeptin and 0.35 mg/mL of APMSF)], and were disrupted with a Potter homogenizer, followed by a slow centrifugation treatment (at 1,500 rpm for 10 minutes at 4°C). Subsequently, the supernatant was collected and treated in an ultracentrifuge (at 100,000 $\times$ g for 1 hour at 4°C), and the precipitated membrane fraction was collected and suspended in a phosphate buffer to store at -20°C. This suspension was used as the immunogen for producing human antibodies.

Normal mice were immunized with the resulting cell membrane fraction, thereby preparing a mouse monoclonal antibody against hLOX-1.

The preparation of monoclonal antibodies was carried out in accordance with the common method described in "Jikken Igaku (supplementary volume), Cell Engineering Handbook" (edited by Toshio Kuroki et al., published by Yodosha Co., Ltd., pp. 66-74, 1992) and "The Introduction to Monoclonal Antibody Experimental Procedure" (Tamie Ando et al., published by Kodansha Ltd., 1991).

Note that the term "anti-LOX-1 antibody" refers to a specific antibody of LOX-1 as an antigen and acts as an antagonist of LOX-1 by an antigen-antibody reaction.

[Example 1: Evaluation of bonding between LOX-1 and procyanidin by ELISA]

The bonding properties between the AP and the LOX-1 of procyanidins with each degree of polymerization prepared in Preparation Examples 1 and 2 were evaluated by a method, which further improved the method of Sato and others (refer to Atherosclerosis, 2008, vol. 200, No. 2, pages 303-309). More specifically, the ELISA plate prepared in Preparation Example 4 and the oxidized LDL prepared in Preparation Example 5 were used to examine as to whether the bonding between LOX-1 and oxidized LDL was inhibited when procyanidins or the like was added to the reaction solution.

First, the oxidized LDL and various procyanidins (including AP) were added to the aforementioned buffer for ELISA measurements, so that their final concentrations were 250 ng/ml and 12.5, 25, 50, 100, 200 or 400 $\mu$g/ml, respectively, and 40 $\mu$l of the respective samples prepared was dispensed into each well of the ELISA plate. After leaving this plate to stand at room temperature for 2 hours, each well was washed five times with PBS. Subsequently, 50 $\mu$l of a diluted solution of anti-human apolipoprotein B antibody (anti Human Apolipoprotein B PEROX, the binding site: PP086) which was diluted 500-fold using a HEPES buffer containing 1% BSA and 2 mM EDTA was dispensed into each well, and was then left to stand at room temperature for 1 hour. After being left to stand, each well was washed five times with PBS, and the color of TMB was developed using the TMB Peroxidase EIA substrate kit (manufactured by Bio-Rad, Product Number: 68-12-2 (A) 2722-84-1 (B)). After the chromogenic reaction was terminated by adding 2M sulfuric acid to each well, the absorbance at 450 nm was measured.

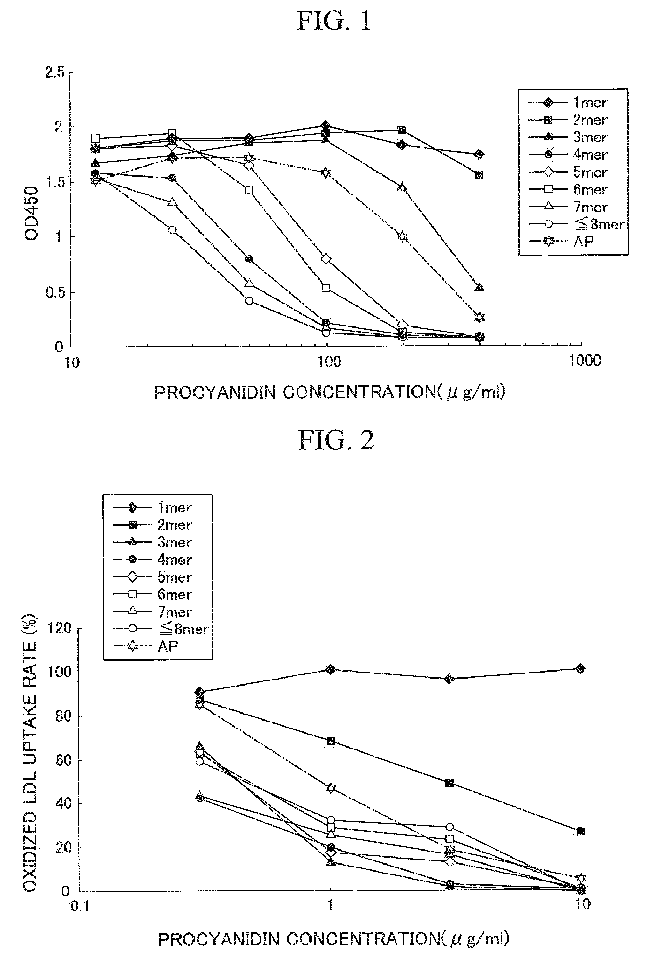

FIG. 1 is a diagram showing the results of absorbance measured at 450 nm (OD450) for procyanidins with each degree of polymerization. In the diagram, the expressions "Xmer" and "$\leq$ 8mer" refer to a procyanidin whose degree of polymerization is X, and octameric or higher polymeric procyanidins, respectively. The lower the absorbance, the less the oxidized LDL bound to LOX-1, which indicates the bonding between LOX-1 and oxidized LDL being inhibited by the procyanidins or AP. As a result, it became clear that AP was inhibiting the bonding between LOX-1 and oxidized LDL, in other words, AP exhibited an LOX-1 inhibiting activity. In addition, because this LOX-1 inhibiting activity was also observed in procyanidins, it was presumed that the LOX-1 inhibiting activity exhibited by AP was mainly due to procyanidins. It was found that trimeric or higher polymeric procyanidins, in particular, exhibited strong LOX-1 inhibiting activities.

[Example 2: Evaluation of uptake inhibition of oxidized LDL by cells expressing LOX-1]

Subsequently, the extent of inhibition of the bonding between LOX-1 and oxidized LDL by procyanidins was evaluated by the amount of incorporation of oxidized LDL into the cells that were expressing LOX-1. More specifically, since the CHO cells that were forced to overexpress human LOX-1 (hereinafter, LOX-1 expressing cells) incorporate oxidized LDL, the effects of procyanidins and AP on the uptake of fluorescently labeled oxidized LDL mediated by the LOX-1 were observed. Note that the TetOn hLOX-1 CHO cells prepared in Preparation Example 7 were used as the LOX-1 expressing cells, and the Dil-labeled oxidized LDL prepared in Preparation Example 6 was used as the fluorescently labeled oxidized LDL, respectively. In addition, the AP and procyanidins with each degree of polymerization prepared in Preparation Examples 1 and 2 were used as AP and procyanidins.

First, 100 μl of the suspension of LOX-1 expressing adjusted with the above culture medium so as to achieve a density of $1 \times 10^5$ cells/ml was dispensed into each well of a 96-well plate (manufactured by Costar, Product Number: 3603). In addition, in order to induce the expression of LOX-1, doxycycline (manufactured by Calbiochem) was added so that the final concentration thereof was 1 μg/ml. This 96-well plate was incubated at 37°C for 24 hours in a $CO_2$ incubator, and each well was then washed with an FBS(-) medium. Note that the FBS(-) medium is a medium prepared by removing only FBS from the aforementioned culture medium.

Subsequently, 100 μl of various procyanidins (including AP) adjusted with the FBS(-) medium so as to achieve a concentration of 0.3, 1, 3, or 10 μg/ml was dispensed into each well, and the 96-well plate was then incubated at 37°C for 1 hour in a $CO_2$ incubator. After washing each well with the FBS(-) medium, 100 μl of the DiI-labeled oxidized LDL adjusted with the FBS(-) medium so as to achieve a concentration of 1 μg/ml was dispensed into each well, and the 96-well plate was then incubated at 37°C for 2 hours in a $CO_2$ incubator. After washing each well twice with the FBS(-) medium, a 10% neutral buffered formalin solution (manufactured by Wako Pure Chemical Industries, Ltd.) was added to each well for cell fixation, and 1 μg/ml of DAPI solution (manufactured by Sigma-Aldrich Co. LLC.) was further added thereto to carry out nuclear staining.

This 96-well plate was observed using the In Cell Analyzer 1000 system (manufactured by GE healthcare) to measure the DiI fluorescence intensity and the number of nuclei per each well. From the measurements, the fluorescence intensity per single cell was determined using the following formula (1).

Formula (1): Fluorescence intensity per single cell = [DiI fluorescence intensity per well]

/ [number of nuclei per well]

Further, the oxidized LDL uptake rate (%) was calculated by the following formula (2). It should be noted that A denotes the fluorescence intensity per single cell in a well where various procyanidins (including AP) were not added, B denotes the fluorescence intensity per single cell in a well where the DiI-labeled oxidized LDL was not added, and X denotes the fluorescence intensity per single cell in a well where various procyanidins (including AP) and the DiI-labeled oxidized LDL were added. Formula (2): Oxidized LDL uptake rate (%) = $(X-B) / (A-B) \times 100$

FIG. 2 is a diagram showing the calculated uptake rate (%) of oxidized LDL for procyanidins with each degree of polymerization. In the diagram, the expressions "Xmer" and "≤ 8mer" are the same as those defined in FIG. 1. As a result, since the oxidized LDL uptake rate was reduced, similar to the results of Example 1, it was confirmed that AP and dimeric or higher polymeric procyanidins inhibit the bonding between LOX-1 and oxidized LDL in a concentration dependent manner. It was found that trimeric or higher polymeric procyanidins, in particular, exhibited higher LOX-1 inhibiting activities than AP at low concentrations. On the other hand, no LOX-1 inhibiting activity was observed in monomeric procyanidins.

It should be noted that unlike other screening methods for arteriosclerosis inhibitors in which whether arteriosclerosis was suppressed or not was used as an indicator, inhibitors for LOX-1 can be selected, by focusing on the LOX-1 involved in the exacerbation of various cardiovascular diseases, through an assay system using the LOX-1 expressing cells and the fluorescently labeled oxidized LDL as in the present example.

[Example 3: Evaluation of uptake inhibition of oxidized LDL by procyanidins derived from other plants]

Procyanidins derived from plants other than apples were also examined in the same manner as to whether they exhibit LOX-1 inhibiting activities. Further, instead of procyanidins, various polyphenols containing a large amount of procyanidins [AP (prepared in Preparation Example 1), polyphenols derived from grape seeds (trade name: Gravinol, manufactured by Kikkoman Corporation), polyphenols derived from peanut seed coats (manufactured by Kisco Ltd.), and polyphenols derived from pine barks (manufactured by Tradepia Corporation)] were used as the measurement samples.

More specifically, measurements were carried out in the same manner as in Example 2 to calculate the oxidized LDL uptake rate (%), with the exception that various polyphenols adjusted with the FBS(-) medium so as to achieve a concentration of 10 μg/ml were used instead of various procyanidins. Note that those to which the DMSO solution was added instead of the respective polyphenols at the same concentration thereof were used as blanks.

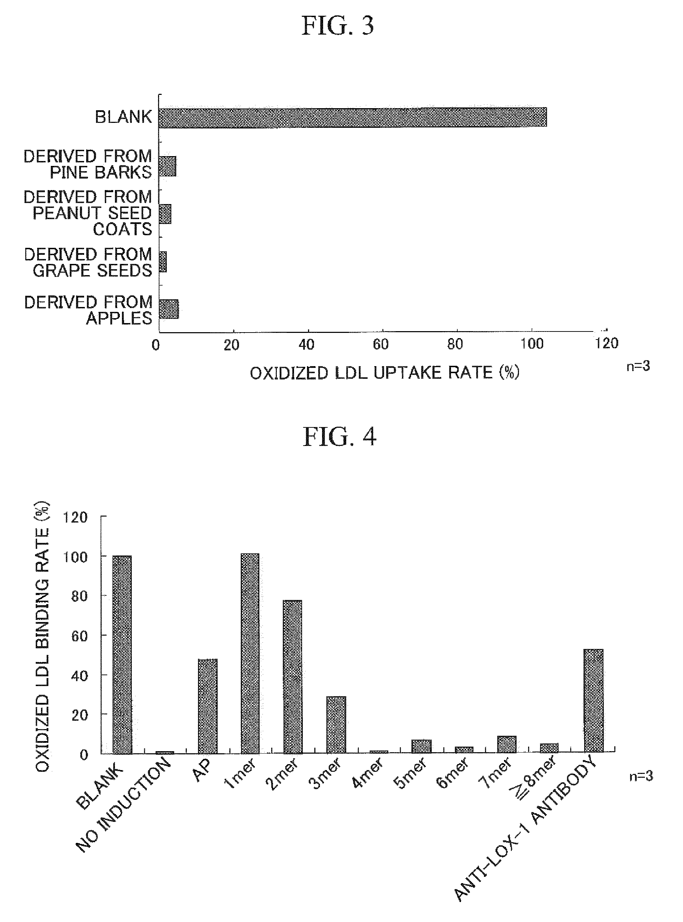

FIG. 3 is a diagram showing the uptake rate (%) of oxidized LDL in blanks or when the respective polyphenols was added. As a result, the activity to inhibit the bonding between oxidized LDL and LOX-1 observed in AP was also observed in a similar manner in the polyphenols derived from pine barks, grape seeds and peanut seed coats, which were other materials containing procyanidins, and thus they were also confirmed to exhibit LOX-1 inhibiting activities.

Because the respective polyphenols contained a large amount of procyanidins as with AP, it is clear from the results of the present example that the trimeric or higher polymeric procyanidins separated and purified from the respective polyphenols would also exhibit LOX-1 inhibiting activities, as with the respective polyphenols.

[Example 4: Evaluation of inhibition of bonding of oxidized LDL by cells expressing LOX-1]

It is said that caveola or clathrin is also involved in the incorporation of oxidized LDL into the cells, in addition to that mediated by LOX-1. Accordingly, in order to evaluate the activity to inhibit the bonding between LOX-1 and oxidized LDL more directly, cells were placed under low temperature conditions so as to suppress endocytosis, thereby evaluating as to whether procyanidins inhibit the bonding between LOX-1 and oxidized LDL under these conditions.
First, 100 $\mu$l of the suspension of LOX-1 expressing cells adjusted with the above culture medium so as to achieve a density of $1 \times 10^5$ cells/ml was dispensed into each well of a 96-well plate (manufactured by Costar, Product Number: 3603). In addition, in order to induce the expression of LOX-1, doxycycline (manufactured by Calbiochem) was added so that the final concentration thereof was 1 $\mu$g/ml. As a control, a well where no doxycycline was added so that no expression was induced was also prepared. This 96-well plate was incubated at 37°C for 24 hours in a $CO_2$ incubator, and each well was then washed with the aforementioned FBS(-) medium cooled to 4°C.
This 96-well plate was left to stand on ice for 30 minutes in a refrigerator. Subsequently, 100 $\mu$l of various procyanidins (including AP) adjusted with the FBS(-) medium cooled to 4°C so as to achieve a concentration of 0.1 $\mu$g/ml was dispensed into each well, and the 96-well plate was then left to stand on ice for 1 hour in a refrigerator. At this time, those received the FBS(-) medium cooled to 4°C, to which DMSO was added instead of various procyanidin at the same concentration thereof, were used as blanks. In addition, those received, instead of various procyanidins, the anti-LOX-1 antibody solution adjusted with the FBS(-) medium cooled to 4°C so as to achieve a concentration of 0.3 $\mu$g/ml were used as positive controls. Further, that prepared in Preparation Example 8 was used as the anti-LOX-1 antibody.
After washing each well with the FBS(-) medium cooled to 4°C, 100 $\mu$l of the DiI-laheled oxidized LDL adjusted with the FBS(-) medium cooled to 4°C so as to achieve a concentration of 1 $\mu$g/ml was dispensed into each well, and the 96-well plate was then left to stand on ice for 1 hour in a refrigerator. After washing each well twice with the FBS(-) medium cooled to 4°C, a 10% neutral buffered formalin solution (manufactured by Wako Pure Chemical Industries, Ltd.) cooled to 4°C was added to each well for cell fixation, and after returning to room temperature, 1 $\mu$g/ml of DAPI solution (manufactured by Sigma-Aldrich Co. LLC.) was further added thereto to carry out nuclear staining.
This 96-well plate was observed using the In Cell Analyzer 1000 system (manufactured by GE healthcare) to measure the DiI fluorescence intensity and the number of nuclei per each well, and the fluorescence intensity per single cell was determined in the same manner as in Example 2.
Further, the oxidized LDL binding rate (%) was calculated by the following formula (3). Note that A, B and X denote the same as those defined in the above formula (2).

$$\text{Formula (3): Oxidized LDL binding rate (\%)} = (X-B) / (A-B) \times 100$$

FIG. 4 is a diagram showing the binding rate (%) of oxidized LDL when various procyanidins were added. In the diagram, the expressions "Xmer" and "$\leq$ 8mer" are the same as those defined in FIG. 1. In addition, "No induction" indicates the results of the well where no doxycycline was added so that no expression was induced. As a result, almost no oxidized LDL was bound in the well where the expression of LOX-1 was not induced. Further, in the well where the anti-LOX-1 antibody known to bind to Lox-1 was added, the binding rate of oxidized LDL was about 53%, thereby confirming that the binding with oxidized LDL was inhibited. From these results, it was verified that the assay system of the present example was capable of measuring the effects of various procyanidins on the bonding between LOX-1 and oxidized LDL.
As shown in FIG. 4, it was confirmed that AP and dimeric or higher polymeric procyanidins inhibit the bonding between LOX-1 and oxidized LDL. It was found that trimeric or higher polymeric procyanidins, in particular, exhibited higher LOX-1 inhibiting activities than AP. On the other hand, no LOX-1 inhibiting activity was observed in monomeric procyanidins.

[Example 5: Evaluation of inhibition of bonding of oxidized LDL by procyanidins with different structures]

The effects of procyanidin structures on the LOX-1 inhibiting activity were examined.
The trimeric procyanidins prepared in Preparation Example 2 were further separated and purified, depending on the structures thereof, by reverse phase chromatography, and the resultant was used to evaluate the inhibition of the binding of oxidized LDL in the same manner as in Example 4.
First, the trimeric procyanidins prepared in Preparation Example 2 were dissolved in water and were then applied to the Inertsil ODS-3 column (manufactured by GL Sciences Inc., 4 μm, φ 20 × 250 mm), and were eluted using 20% methanol as the mobile phase under the conditions of a flow rate of 12.0 ml/min and a column temperature of 40°C. The absorbance of eluate at 280 nm was measured using a UV detector, thereby detecting three peaks between 14 and 24 minutes, in terms of retention time (elution time), and a single peak between 40 and 55 minutes.

The fractions for each peak were purified once again. First, the fractions for the retention time of between 14 and 24 minutes were concentrated, freeze-dried, and were then dissolved in water once again, applied to the Inertsil ODS-3 column (manufactured by GL Sciences Inc., 4 μm, φ 20 × 250 mm), and were then eluted using 15% methanol as the mobile phase under the conditions of a flow rate of 12.0 ml/min and a column temperature of 40°C. The absorbance of eluate at 280 nm was measured using a UV detector, thereby detecting a single peak (peak 1) between 33 and 37 minutes in terms of retention time, a single peak (peak 2) between 40 and 44 minutes, and a single peak (peak 3) between 48 and 58 minutes, respectively.

Separate from this, the fraction obtained in the first reverse phase chromatography for the retention time of between 40 and 55 minutes was concentrated, freeze-dried, and was then dissolved in water once again, applied to the Inertsil ODS-3 column (manufactured by GL Sciences Inc., 4 μm, φ 20 × 250 mm), and was then eluted using 20% methanol as the mobile phase under the conditions of a flow rate of 12.0 ml/min and a column temperature of 40°C. The absorbance of eluate at 280 nm was measured using a UV detector, thereby detecting a single peak (peak 4) between 40 and 55 minutes in terms of retention time.

Each of these fractions for the peaks 1 to 4 was concentrated, freeze-dried, and was then suspended in the reagent grade DMSO for biochemical analysis (manufactured by Wako Pure Chemical Industries, Ltd.) for use. When the inhibition of the bonding of oxidized LDL was measured, the suspension was diluted with ultrapure water for use, where appropriate, so that the final concentration of DMSO in the reaction solution was 1% or less.

The structures for the trimeric procyanidins of each peak were examined to find that they were the compounds indicated in Table 1 (J. Agric. Food Chem., 2003, vol. 51, pp. 3806-3813). In the table, the expression "epi" denotes an epicatechin, "cat" denotes a catechin, and "(4β → 8)" describes a structure condensed between the carbon atoms from the 4th to 8th positions.

[Table 1]

| peak 1 | epi (4β → 8) epi (4β → 8) cat |
| --- | --- |
| peak 2 | epi (4β → 6) epi (4β → 8) cat |
| peak 3 | epi (4β → 6) epi (4β → 8) epi |
| peak 4 | epi (4β → 8) epi (4β → 8) epi |

Subsequently, the effects of these four types of trimeric procyanidins on the inhibition of the bonding between LOX-1 and oxidized LDL were evaluated. More specifically, the evaluation was carried out in the same manner as in Example 4, with the exception that various trimeric procyanidins adjusted with the FBS(-) medium cooled to 4°C so as to achieve a concentration of 0.1 μg/ml were used.

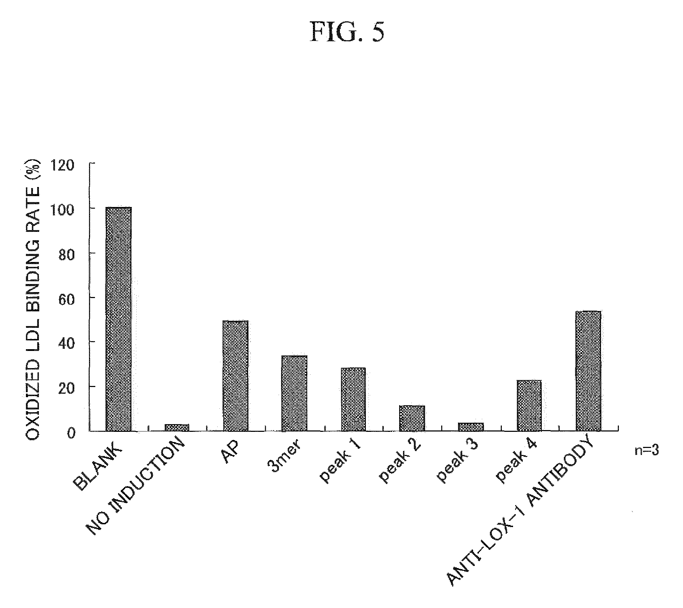

FIG. 5 is a diagram showing the binding rate (%) of oxidized LDL in blanks or when four types of trimeric procyanidins with different structures were added. In the diagram, the expressions "3mer" and "No induction" are the same as those defined in FIG. 4. As a result, all four types of trimeric procyanidins purified based on the different structures (represented by peaks 1 to 4) exhibited high LOX-1 inhibiting activities. Since the trimeric procyanidins represented by peaks 2 and 3, in particular, exhibited stronger LOX-1 inhibiting activities than the trimeric procyanidins represented by peaks 1 and 4, it was suggested that the procyanidins having a structure in which a condensation site was present between the carbon atoms from the 4th to 6th positions of Flavan-3-ol or the procyanidins terminated with epicatechin would exhibit a relatively strong LOX-1 inhibiting activity.

It should be noted that all of peaks 1 to 4 exhibited stronger LOX-1 inhibiting activities than the trimeric procyanidins prior to the separation and purification (expressed as "3mer" in FIG. 5). It is thought that this is because the trimeric procyanidins prior to the separation and purification also contained some kinds of impurities, in addition to the four types of trimeric procyanidins listed in Table 1. In fact, in the first reverse phase chromatography, the absorbance at 280 nm was higher than the baseline, although no clear peak was detected prior to the peak 1, between the peak 3 and peak 4, or after the peak 4. Accordingly, it is suggested that some unknown compounds were eluted, and that some kinds of impurities were contained in addition to procyanidins.

[Example 6: Evaluation of bonding between LOX-1 and procyanidin by BIACORE measurement]

The direct bonding of procyanidins and LOX-1 was evaluated using the BIACORE system. The ex-hLOX-1 protein produced in Preparation Example 3 was used as the LOX-1 protein. On the other hand, as procyanidins, procyanidins with each degree of polymerization (from monomers to heptamers) prepared in Preparation Example 2, and epi $(4\beta \to 6)$ epi $(4\beta \to 8)$ epi (hereafter, sometimes referred to as "peak 3 trimers") separated and purified as the peak 3 from the trimeric procyanidins in Example 5 were used.

LOX-1 was fixed on a sensor chip, and the BIACORE measurement was carried out using the procyanidins as the liquid phase. The BIACORE measurements and analyses were conducted using the BIACORE 2000 (GE Healthcare). In addition, the sensor chip CM5 was used as a sensor chip, and the ex-hLOX-1 protein was immobilized on the sensor chip by employing the amine coupling method. A HEPES solution (consisted of 10 mM HEPES, 150 mM NaCl and 2 mM $CaCl_2$) was degassed and used as the running buffer.

Among the procyanidins made to flow through the chip, monomeric to heptameric procyanidins were each adjusted to 20 $\mu$M using the running buffer and used for the measurement. On the other hand, the peak 3 trimers were each adjusted to a concentration of 20, 10, 5, 2.5 or 1.25 $\mu$M using the same running buffer and used for the measurement.

In addition, when the procyanidins were reacted in the sensor chip, the flow rate was set to 20 $\mu$l/min, and the dissociation constant was measured with 120 seconds of binding and 120 seconds of dissociation. On the other hand, during regeneration, the bonding between procyanidin and LOX-1 was dissociated by causing 50 mM NaOH to flow for 5 seconds at a flow rate of 60 $\mu$l/min.

The results of the dissociation constant measured for each procyanidin are indicated in Table 2. As a result, the binding with LOX-1 was observed in procyanidins except monomeric procyanidins. Moreover, the dissociation constant reduces as the degree of polymerization increases, thereby revealing the strong bond with LOX-1. These results are consistent with the results measured using the ELISA method or the cells expressing LOX-1. From these results, it can be concluded that procyanidins bind directly to LOX-1 and inhibit the bonding between oxidized LDL and LOX-1.

[Table 2]

| Procyanidin | Dissociation Constant |
| --- | --- |
| Monomer | No bonding |
| Dimer | $3.6 \times 10^{-5}$ |
| Trimer | $5.2 \times 10^{-6}$ |
| Tetramer | $3.5 \times 10^{-6}$ |
| Pentamer | $2.3 \times 10^{-6}$ |
| Hexamer | $2.0 \times 10^{-6}$ |
| Heptamer | $1.3 \times 10^{-6}$ |
| peak 3 trimer | $3.8 \times 10^{-6}$ |

[Example 7: Evaluation of effects on arteriosclerosis prevention by procyanidins]

The effects of procyanidins on arteriosclerosis were observed using the stroke-prone spontaneously hypertensive rat (SHR-SP) system for evaluating the number of mesenteric lipid deposits that has been used as an evaluation system for arteriosclerosis.

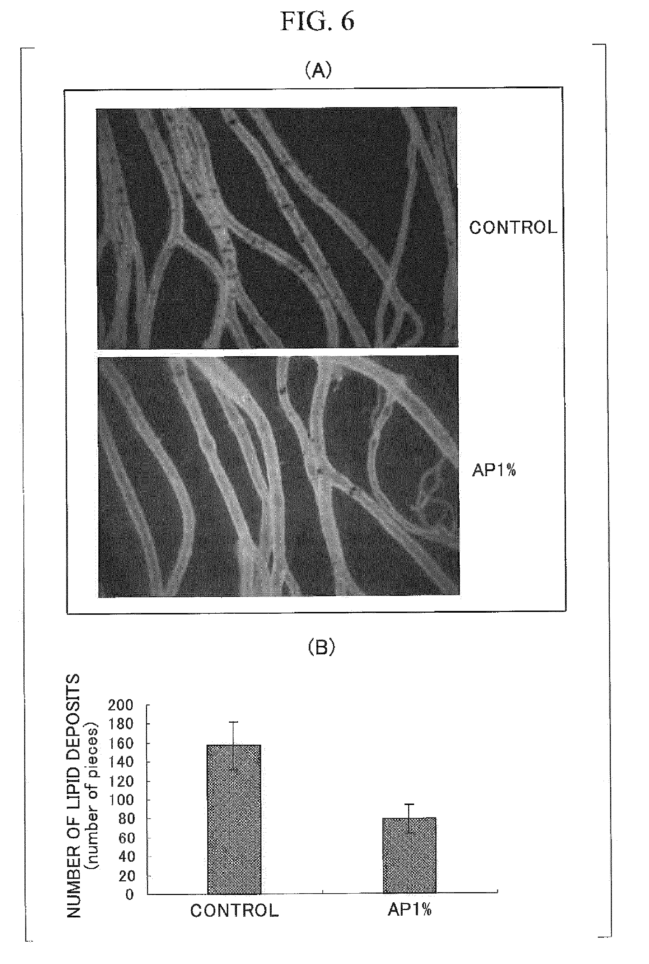

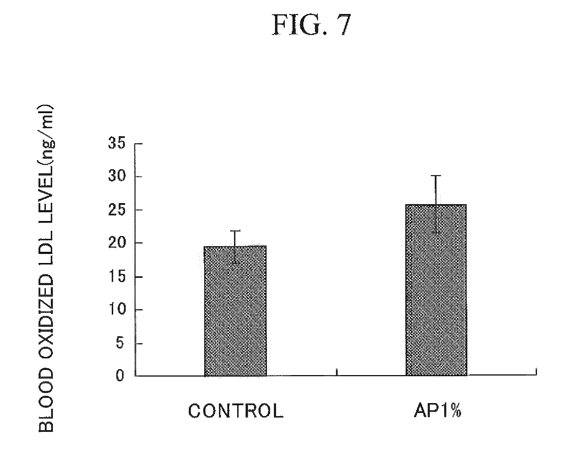

First, eight 8-week-old SHR-SP rats were given free access to food and drink and fed with a high fat diet containing 1% of AP and with physiological saline for 2 weeks (1% AP group). On the other hand, as a control, eight 8-week-old SHR-SP rats were given free access to food and drink in the same manner and fed with a high fat diet containing no AP and with physiological saline for 2 weeks (control group). After two weeks, serum was collected, and the level of oxidized LDL in the blood was measured by ELISA, while using the oxidized LDL prepared in the same manner as in Preparation Example 5 for the calibration curve. Furthermore, mesenteric artery was collected from each rat through dissection, and the lipids in the mesenteric arteries were stained with Oil Red O (dye) to measure the number of lipid deposits. FIG. 6 (A) is a stained image of lipids in the mesenteric arteries of rats, and FIG. 6(B) is a diagram showing the number of lipid deposits observed in the mesenteric arteries of rats. In FIG. 6(A), black circular dots in the arteries (appearing as white tubes in the diagram) correspond to the site of lipid deposition. In addition, FIG. 7 is a diagram showing the level of oxidized LDL in the rat blood.

As a result, as shown in FIG. 6, a significant decrease in the number of lipid deposition was observed in the 1% AP group, as compared to the control group. Accordingly, it was confirmed that arteriosclerosis may be prevented through the ingestion ofAP. On the other hand, as shown in FIG. 7, no significant difference in the blood oxidized LDL level was observed between the two groups. For this reason, it was confirmed that AP inhibited the uptake of oxidized LDL into cells by inhibiting the bonding between LOX-1 and oxidized LDL, rather than suppressing the formation of oxidized LDL. That is, it was verified that AP exhibits an LOX-1 inhibiting activity not only *in vitro,* but also *in vivo.*

In other words, from these results, it is apparent that the onset of cardiovascular diseases such as arteriosclerosis can be suppressed through the ingestion of polyphenols containing trimeric or higher polymeric procyanidins.

[Industrial Applicability]

The drug for inhibiting lectin-like oxidized LDL receptors and the drug for preventing arteriosclerosis, which employs the former drug, according to the present invention exhibit highly superior LOX-1 inhibiting activities and are also highly safe, and thus can be taken continuously. For this reason, they can be used in the field of producing drugs for the treatment and prevention of diseases where LOX-1 acts as an aggravating factor, such as inflammation, arteriosclerosis, thrombosis, myocardial infarction, and vascular restenosis following catheter treatments.

**Claims**

1. A drug for inhibiting a lectin-like oxidized LDL receptor comprising a trimeric or higher polymeric procyanidin as an active ingredient.

2. The drug for inhibiting a lectin-like oxidized LDL receptor according to Claim 1,
   wherein the procyanidin is derived from a plant material selected from the group consisting of apples, grape seeds, peanut seed coats and pine barks.

3. A drug for preventing arteriosclerosis comprising a trimeric or higher polymeric procyanidin as an active ingredient, and having an activity to inhibit a lectin-like oxidized LDL receptor.

4. The drug for preventing arteriosclerosis according to Claim 3,
   wherein the procyanidin is derived from a plant material selected from the group consisting of apples, grape seeds, peanut seed coats and pine barks.

5. A drug for inhibiting a lectin-like oxidized LDL receptor comprising a procyanidin having a structure in which at least one condensation site is present between carbon atoms from 4th to 6th positions of Flavan-3-ol as an active ingredient.

6. A drug for inhibiting a lectin-like oxidized LDL receptor comprising a procyanidin terminated with epicatechin as an active ingredient.

# FIG. 1

# FIG. 2

# FIG. 3

# FIG. 4

FIG. 5

# FIG. 6

(A)

CONTROL

AP1%

(B)

NUMBER OF LIPID DEPOSITS (number of pieces)

CONTROL    AP1%

FIG. 7

<div align="center">

**INTERNATIONAL SEARCH REPORT**

</div>

| | International application No. |
|---|---|
| | PCT/JP2010/004177 |

**A. CLASSIFICATION OF SUBJECT MATTER**
*C07D311/62*(2006.01)i, *A61K31/352*(2006.01)i, *A61P9/10*(2006.01)i, *A61P43/00*
(2006.01)i

According to International Patent Classification (IPC) or to both national classification and IPC

**B. FIELDS SEARCHED**

Minimum documentation searched (classification system followed by classification symbols)
C07D311/62, A61K31/352, A61P9/10, A61P43/00

Documentation searched other than minimum documentation to the extent that such documents are included in the fields searched
| | | | |
|---|---|---|---|
| Jitsuyo Shinan Koho | 1922-1996 | Jitsuyo Shinan Toroku Koho | 1996-2010 |
| Kokai Jitsuyo Shinan Koho | 1971-2010 | Toroku Jitsuyo Shinan Koho | 1994-2010 |

Electronic data base consulted during the international search (name of data base and, where practicable, search terms used)
CAplus/REGISTRY/MEDLINE/EMBASE/BIOSIS(STN),
JSTPlus/JMEDPlus/JST7580(JDreamII)

**C. DOCUMENTS CONSIDERED TO BE RELEVANT**

| Category* | Citation of document, with indication, where appropriate, of the relevant passages | Relevant to claim No. |
|---|---|---|
| X | FUHRMAN, B. et al, Grape powder polyphenols attenuate atherosclerosis development in apolipoprotein E deficient (E0) mice and reduce macrophage atherogenicity, The Journal of nutrition, 2005, Vol.135, No.4, pp.722-728 | 1-6 |
| X | JANISCH, K.M. et al, Simulated digestion of Vitis vinifera seed powder: Polyphenolic content and antioxidant properties, Journal of Agricultural and Food Chemistry, 2006, Vol.54, No.13, pp.4839-4848 | 1-6 |
| X | LEITE, D.S. et al, Antioxidant protection of low density lipoprotein by procyanidins: structure/activity relationships, Biochemical Pharmacology, 2003, Vol.66, No.6, pp.947-954 | 1-6 |

| ☒ | Further documents are listed in the continuation of Box C. | ☐ | See patent family annex. |
|---|---|---|---|

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 13 July, 2010 (13.07.10) | 20 July, 2010 (20.07.10) |

| Name and mailing address of the ISA/ | Authorized officer |
|---|---|
| Japanese Patent Office | |
| Facsimile No. | Telephone No. |

Form PCT/ISA/210 (second sheet) (July 2009)

## INTERNATIONAL SEARCH REPORT

| International application No. |
| --- |
| PCT/JP2010/004177 |

C (Continuation).   DOCUMENTS CONSIDERED TO BE RELEVANT

| Category* | Citation of document, with indication, where appropriate, of the relevant passages | Relevant to claim No. |
| --- | --- | --- |
| X | TEISSEDRE, P.L. et al, Inhibition of in vitro human LDL oxidation by phenolic antioxidants from grapes and wines, Journal of the Science of Food and Agriculture, 1996, Vol.70, No.1, pp.55-61 | 1-6 |
| X | JP 2006-182706 A  (Kikkoman Corp.), 13 July 2006 (13.07.2006), entire text; particularly, claim 5; paragraph [0049] (Family: none) | 1-6 |
| X | JP 8-225453 A  (Suntory Ltd.), 03 September 1996 (03.09.1996), entire text; particularly, claims; examples & US 5607965 A           & EP 713706 A3 | 1-6 |
| X | JP 2006-193502 A  (Asahi Breweries, Ltd.), 27 July 2006 (27.07.2006), entire text; particularly, claims; paragraph [0007]; examples & WO 2006/064761 A1 | 1-6 |
| X | JP 2005-23032 A  (Toyo Shinyaku Co., Ltd.), 27 January 2005 (27.01.2005), entire text; particularly, claims; paragraph [0028]; examples (Family: none) | 1-6 |
| X | JP 2003-146898 A  (Toyo Shinyaku Co., Ltd.), 21 May 2003 (21.05.2003), entire text; particularly, claims; paragraphs [0010], [0025] to [0031] (Family: none) | 1-6 |
| X | JP 2009-120565 A  (Nitto Best Corp.), 04 June 2009 (04.06.2009), entire text; particularly, claims; paragraph [0002]; examples (Family: none) | 1-6 |
| X | JP 2008-156265 A  (Yamagata-Ken), 10 July 2008 (10.07.2008), entire text; particularly, claims; paragraph [0003]; examples (Family: none) | 1-6 |
| X | JP 2008-88131 A  (Seishin Enterprise Co., Ltd.), 17 April 2008 (17.04.2008), entire text; particularly, claims; paragraph [0009]; examples; fig. 1 (Family: none) | 1-6 |

Form PCT/ISA/210 (continuation of second sheet) (July 2009)

**EP 2 447 257 A1**

**INTERNATIONAL SEARCH REPORT**

| International application No. |
|---|
| PCT/JP2010/004177 |

C (Continuation). DOCUMENTS CONSIDERED TO BE RELEVANT

| Category* | Citation of document, with indication, where appropriate, of the relevant passages | Relevant to claim No. |
|---|---|---|
| X | JP 2009-1531 A (Meiji Yakuhin Co., Ltd.), 08 January 2009 (08.01.2009), entire text; particularly, claims; examples (Family: none) | 1,3,5,6 |
| X | WO 2005/116005 A1 (Suntory Ltd.), 08 December 2005 (08.12.2005), entire text; particularly, claims; paragraph [0036]; examples & US 2008/0275258 A1 & EP 1754702 A1 | 1,3,5,6 |
| X | JP 2003-231684 A (Ichimaru Pharcos Co., Ltd.), 19 August 2003 (19.08.2003), entire text; particularly, claims; examples; fig. 1 to 3 (Family: none) | 1,3,6 |
| X | WO 2001/078529 A1 (Mars, Inc.), 25 October 2001 (25.10.2001), entire text; particularly, claims(claim 16); examples & JP 2003-530410 A & US 2002/0018807 A1 & EP 1274319 A1 | 1,3 |
| X | JP 9-291039 A (Suntory Ltd.), 11 November 1997 (11.11.1997), entire text; particularly, claims; examples; paragraph [0039] & WO 1997/023210 A1 & US 6294190 B1 & EP 815857 A1 | 1,3,6 |
| P,X | WO 2010/024393 A1 (Suntory Holdings Ltd.), 04 March 2010 (04.03.2010), entire text; particularly, claims; example 2; paragraph [0002] (Family: none) | 1,3,6 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2009148658 A
- JP 2006193502 A
- JP 2005023032 A
- JP 2007297381 A
- JP 2007320956 A

### Non-patent literature cited in the description

- **Sugiyama.** *Journal of Agricultural and Food Chemistry,* 2007, vol. 55, 4604-4609
- *Atherosclerosis,* 2008, vol. 200 (2), 303-309
- **M. Olinishikameyama.** *Mass Spectrometry,* 1997, vol. 11, 31-36
- *Nature,* 1997, vol. 386, 73-77
- Jikken Igaku. Yodosha Co., Ltd, 1992, 66-74
- **Tamie Ando et al.** The Introduction to Monoclonal Antibody Experimental Procedure. Kodansha Ltd, 1991
- *J. Agric. Food Chem.,* 2003, vol. 51, 3806-3813